# EUROPÄISCHE PATENTSCHRIFT

(11) **EP 1 562 476 B1**
(45) Veröffentlichungstag und Bekanntmachung des Hinweises auf die Patenterteilung: **19.08.2009**
(21) Anmeldenummer: 03769017.9
(22) Anmeldetag: 09.10.2003
(51) Int. Cl.: A61B 5/103

(54) **VORRICHTUNG ZUM ERFASSEN VON WIRBELVERSCHIEBUNGEN**
DEVICE FOR DETECTING VERTEBRAL DISPLACEMENTS
DISPOSITIF POUR DETECTER DES DEPLACEMENTS DE VERTEBRES

(30) Priorität: 31.10.2002 AT 16462002
(43) Veröffentlichungstag der Anmeldung: 17.08.2005
(73) Patentinhaber: Leopold, Horst, 4060 Leonding (AT)
(72) Erfinder: Leopold, Horst, 4060 Leonding (AT)
(74) Vertreter: Hübscher, Helmut
(86) Internationale Anmeldenummer: PCT/AT2003/000303
(87) Internationale Veröffentlichungsnummer: WO 2004/039259

(56) Entgegenhaltungen:
- EP-A- 0 144 528
- DE-A- 19 833 568
- US-A- 889 224
- US-A- 2 295 447
- US-A1- 2002 049 393

## Beschreibung

Die Erfindung bezieht sich auf eine Vorrichtung zum Erfassen von Wirbelverschiebungen mit einer Meßeinrichtung, die eine Führungsschiene für quer zur Führungsschiene verlaufende, in Richtung der Führungsschiene verstellbar gelagerte Meßfühler zur Abstandsmessung der Wirbelsäule eines Patienten von einer durch die Meßeinrichtung bestimmten Bezugsfläche umfaßt.

Um Wirbelverschiebungen beispielsweise aufgrund einer Spondylolyse nicht nur von Hand aus zu erfühlen, sondern unabhängig vom Geschick des Untersuchenden erfassen und dokumentieren zu können, werden im allgemeinen Röntgenaufnahmen vom jeweils betroffenen Abschnitt der Wirbelsäule eines Patienten mit Strahlengängen aus unterschiedlichen Richtungen gemacht, was nicht nur einen erheblichen apparativen Aufwand, sondern auch eine entsprechende Strahlenbelastung des Patienten mit sich bringt.

Zur Erfassung des Verlaufes einer Wirbelsäule in deren Längsrichtung ist es bereits bekannt (US 889 224 A), eine stehende Führungsschiene vorzusehen, in der in einem regelmäßigen Abstand voneinander Längsschlitze zur Aufnahme von quer zur Führungsschiene verlaufenden Meßfühlern gelagert sind, die eine Maßeinteilung aufweisen und nach einer Einstellung innerhalb der Längsschlitze der Führungsschiene zur Anpassung an den Wirbelabstand gegen die Wirbelsäule eines stehenden, mit dem Rücken der Führungsschiene zugekehrten Patienten vorgeschoben werden können, so daß anhand des Verschiebeweges der Meßfühler der Abstand der Wirbelsäule von einer durch die Führungsschiene bestimmten Bezugsfläche gemessen und damit der Verlauf der Wirbelsäule erfaßt werden kann. Diese bekannte Meßeinrichtung eignet sich allerdings nur bedingt zur Erfassung von Wirbelverschiebungen, weil der Verlauf der Wirbelsäule bei unterschiedlichen Wirbelsäulenkrümmungen nicht genau genug erfaßt werden kann.

Der Erfindung liegt somit die Aufgabe zugrunde, eine Vorrichtung zu schaffen, die mit einfachen konstruktiven Mitteln eine ausreichend genaue Erfassung von Wirbelverschiebungen erlaubt, und zwar unabhängig von der Geschicklichkeit des die Vorrichtung Benutzenden und ohne Belastung für den zu untersuchenden Patienten.

Ausgehend von einer Vorrichtung der eingangs geschilderten Art löst die Erfindung die gestellte Aufgabe dadurch, daß die Meßeinrichtung Abstandhalter zum Aufsetzen der Meßeinrichtung auf die Wirbelsäule im Rückenbereich des Patienten aufweist, daß die durch die Abstandhalter bestimmte Aufsetzfläche der Meßeinrichtung die Bezugsfläche für die Abstandsmessung der einzelnen Wirbel bildet und daß die Meßfühler aus einem an der Führungsschiene festklemmbaren Träger, einem auf dem Träger gegen die Kraft einer Rückstellfeder quer zur Aufsetzfläche verschiebbaren Meßkopf und aus einem Weggeber für den Verschiebeweg des Meßkopfes bestehen.

Nach einer Einstellung des gegenseitigen Abstandes der Meßfühler entsprechend dem Wirbelabstand des zu untersuchenden Patienten wird dann beim eigentlichen Meßvorgang über die Meßfühler der Abstand der einzelnen Wirbel von einer Bezugsfläche gemessen, die in einfacher Weise durch die Abstandhalter bestimmt wird, über die die Meßeinrichtung im Rückenbereich auf die Wirbelsäule des Patienten aufgesetzt wird. Der durch die Meßfühler erfaßbare Abstand der einzelnen Wirbel von der die Bezugsfläche bildenden Aufsetzfläche der Meßeinrichtung läßt einen eindeutigen Rückschluß auf die Verschiebung von Wirbeln aus ihrer normalen Lage zu, wenn die Wirbellage zur Bestimmung einer Spondylolisthesis in an sich bekannter Weise bei unbelasteter und belasteter Wirbelsäule bestimmt wird. Dabei ergeben sich besonders einfache Konstruktionsverhältnisse, weil die Meßköpfe der Meßfühler, die vorab entsprechend den Abständen der vorzugsweise auf dem Rücken der Patienten markierten Meßpunkte eingestellt wurden, beim Aufsetzen der Abstandhalter der Meßeinrichtung auf den Rücken des Patienten gegen die Kraft der Rückstellfedern verschoben werden, wobei die Relatiwerschiebung der einzelnen Meßköpfe die unterschiedlichen Abstände der Meßpunkte von der durch die Aufsetzfläche der Abstandhalter bestimmten Bezugsfläche widerspiegeln. Die Rückstellfedern sichern dabei ein Anliegen der Meßköpfe an den Meßstellen mit einer vorgegebenen Beaufschlagungskraft. Die an der Führungsschiene festklemmbaren Träger, die eine Verschiebeführung für die Meßköpfe bilden, erlauben eine einfache Verlagerung der Meßfühler entlang der Führungsschiene, weil zu diesem Zweck lediglich die Klemmung der Träger zu lösen ist, was beim Einsatz von federbelasteten Klemmeinrichtungen besonders einfache Handhabungsbedingungen schafft.

Im allgemeinen genügt zur Erfassung der Spondylolisthesis die Messung des Abstandes der einzelnen Wirbel von der durch die Aufsetzfläche gegebenen Bezugsfläche durch die Meßfühler, so daß ein Bezug dieser Meßgrößen auf den gegenseitigen Wirbelabstand entfallen kann. Soll zusätzlich der Abstand der vermessenen Lagepunkte der Wirbel in Längsrichtung der Wirbelsäule festgehalten werden, so braucht lediglich der Führungsschiene der Meßeinrichtung ein Lagegeber für die Meßfühler zugeordnet zu werden, weil ja der gegenseitigen Abstände der Meßfühler in Richtung der Führungsschiene den Meßpunktabständen entsprechen.

Um die über die Meßfühler ermittelten Meßwerte für die Diagnose einer Spondylolisthesis vorteilhaft nützen zu können, können die Meßfühler bzw. der Lagegeber an eine mit einer Anzeige- und Ausgabeeinheit verbundene Auswerteschaltung angeschlossen werden, über die die Meßwerte entsprechend verarbeitet werden, um das Untersuchungsergebnis beispielsweise in Form einer Grafik zu veranschaulichen und für spätere Vergleiche zu dokumentieren.

In der Zeichnung ist der Erfindungsgegenstand beispielsweise dargestellt. Es zeigen:

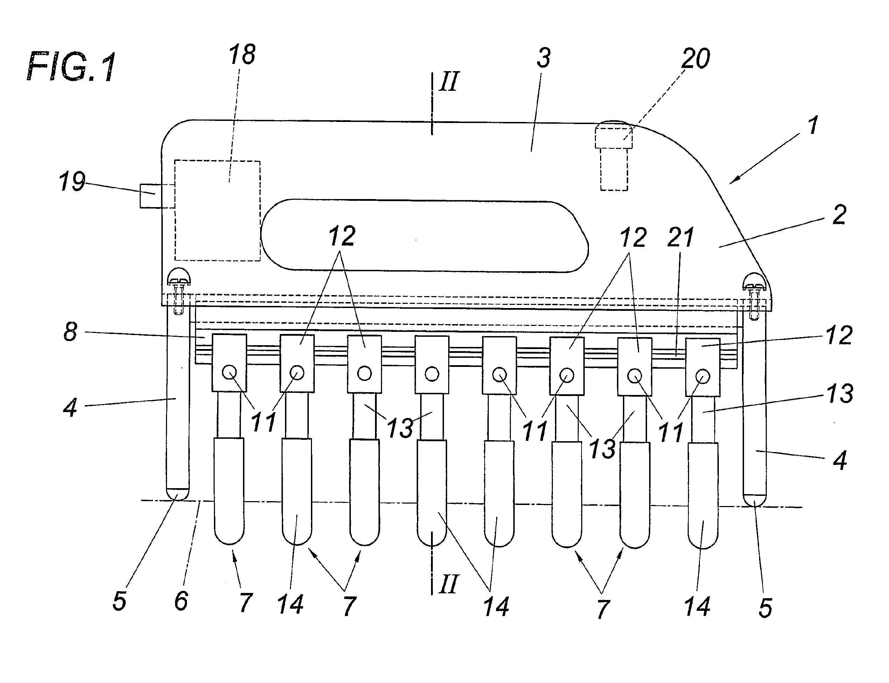

Fig. 1 eine erfindungsgemäße Vorrichtung zum Erfassen von Wirbelverschiebungen in einer vereinfachten Seitenansicht und

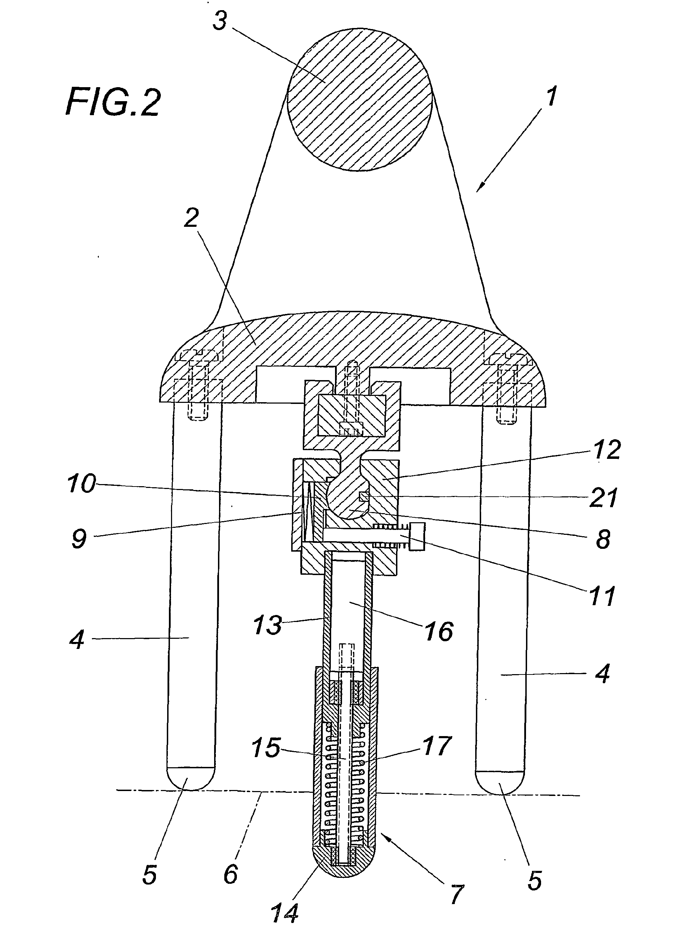

Fig. 2 diese Vorrichtung in einem Schnitt nach der Linie II-II der Fig. 1 in einem größeren Maßstab.

Gemäß dem dargestellten Ausführungsbeispiel wird die Vorrichtung zum Erfassen von Wirbelverschiebungen durch eine Meßeinrichtung 1 gebildet, die ein Gestell 2 mit einem Handgriff 3 zur Handhabung der Meßeinrichtung 1 aufweist. Am Gestell 2 sind bolzenartige Abstandhalter 4 befestigt, die mit ihren Aufsetzenden 5 eine strichpunktiert angedeutete Aufsetzfläche 6 als Bezugsfläche für Meßfühler 7 bestimmen. Diese Meßfühler 7 sind an einer am Gestell 2 befestigten Führungsschiene 8 angeklemmt, und zwar mit Hilfe einer durch eine Klemmfeder 9 belasteten Klemmbacke 10, die über einen Entriegelungshebel 11 gegen die Kraft der Klemmfeder 9 gelöst werden kann, so daß die Meßfühler 7 entlang der Führungsschiene 8 stufenlos verlagert werden können.

Die Meßfühler 7 selbst weisen einen an der Klemmhalterung 12 für die Führungsschiene 8 befestigten Träger 13 für einen auf ihm axial verschiebbaren Meßkopf 14 auf. Der hülsenförmige Meßkopf 14 ist mit einem koaxialen Meßstift 15 verbunden, der in den als Hohlkörper ausgebildeten Träger 13 ragt und mit einem elektronischen Weggeber 16 für den Verschiebeweg des Meßkopfes 14 zusammenwirkt. Der Meßkopf 14 wird durch eine Rückstellfeder 17 in einer anschlagbegrenzten Ausgangsstellung gehalten. Die Weggeber 16 sind an eine elektronische Steuereinrichtung 18 angeschlossen, die Speicher für Meßdaten aufweist. Diese Speicher können über eine Steckverbindung 19 an eine Auswerteschaltung ausgelesen werden, wenn die Steuereinrichtung 18 nicht selbst eine solche Auswerteschaltung umfaßt. Zur Anzeige und Ausgabe der verarbeiteten Meßdaten ist die Auswerteschaltung mit einer Anzeige- und Ausgabeeinheit verbunden. Die Abfrage der Meßwerte und deren Einlesung in die Meßdatenspeicher der Steuereinrichtung 18 erfolgt über einen Tastenschalter 20 im Bereich des Handgriffes 3.

Zum Erfassen von Wirbelverschiebungen werden zunächst durch die zu überprüfenden Wirbel vorgegebene Meßpunkte am Rücken eines Patienten markiert, um dann die Meßfühler 7 entsprechend dem gegenseitigen Abstand der markierten Meßpunkte in Längsrichtung der Wirbelsäule entlang der Führungsschiene 8 zu verteilen, bevor die Meßeinrichtung 1 über den Handgriff 3 so auf den Rücken des Patienten aufgesetzt wird, daß die Meßfühler 7 im Bereich der markierten Meßpunkte zu liegen kommen. Die Meßköpfe 14 werden dabei entlang der Träger 13 gegen die Kraft der Rückstellfedern 17 verschoben, bis die Abstandhalter 4 auf dem Rücken aufsetzen. In dieser Aufsetzlage der Meßeinrichtung 1 werden die Meßwerte der Meßfühler 7 abgerufen und in die Meßdatenspeicher der elektrischen Steuereinrichtung 18 zur nachfolgenden Auswertung eingelesen. Die jeweilige Verschiebelage der Meßköpfe 14 gegenüber der durch die Abstandhalter 4 bestimmten Aufsetzfläche 6 zeigt den jeweiligen Abstand der durch die Wirbel vorgegebenen Meßpunkte gegenüber der Aufsetzfläche 6 an, so daß der Verschiebeweg der Meßköpfe 14 als Maß für die Lage der einzelnen Wirbel dienen kann.

Soll zusätzlich zum Verschiebeweg der einzelnen Meßköpfe 14 der gegenseitige Abstand der Meßfühler 7 entlang der Führungsschiene 8 erfaßt werden, so kann der Führungsschiene 8 ein Lagegeber 21 für die Meßfühler 7 zugeordnet werden, so daß über den Lagegeber 21 entsprechende Meßwerte an die Steuereinrichtung 18 übergeben werden können.

## Patentansprüche

1. Vorrichtung zum Erfassen von Wirbelverschiebungen mit einer Meßeinrichtung (1), die eine Führungsschiene (8) für quer zur Führungsschiene (8) verlaufende, in Richtung der Führungsschiene (8) verstellbar gelagerte Meßfühler (7) zur Abstandsmessung der Wirbelsäule eines Patienten von einer durch die Meßeinrichtung (1) bestimmten Bezugsfläche umfaßt, **dadurch gekennzeichnet, daß** die Meßeinrichtung (1) Abstandhalter (4) zum Aufsetzen der Meßeinrichtung (1) auf die Wirbelsäule im Rückenbereich des Patienten aufweist, daß die durch die Abstandhalter (4) bestimmte Aufsetzfläche (6) der Meßeinrichtung (1) die Bezugsfläche für die Abstandsmessung der einzelnen Wirbel bildet und daß die Meßfühler (7) aus einem an der Führungsschiene (8) festklemmbaren Träger (13), einem auf dem Träger (13) gegen die Kraft einer Rückstellfeder (17) quer zur Aufsetzfläche (6) verschiebbaren Meßkopf (14) und aus einem Weggeber (16) für den Verschiebeweg des Meßkopfes (14) bestehen.

2. Vorrichtung nach Anspruch 1, **dadurch gekennzeichnet, daß** der Führungsschiene (8) ein Lagegeber (21) für die Meßfühler (7) zugeordnet ist.

3. Vorrichtung nach Anspruch 1 oder 2, **dadurch gekennzeichnet, daß** die Meßfühler (7) bzw, der Lagegeber (21) an eine mit einer Anzeige- und Ausgabeeinheit verbundene Auswerteschaltung anschließbar ist.

## Claims

1. Device for detecting vertebral displacements by means of a measuring device (1) which comprises a guide rail (8) for measuring sensors (7) which extend transversely with respect to the guide rail (8) and are adjustably mounted in the direction of the guide rail (8), to measure the spacing of the spinal column of a patient from a reference surface determined by the measuring device (1), **characterised in that** the measuring device (1) comprises spacers (4) for attaching the measuring device (1) onto the spinal column in the region of the patient's back, the attachment surface (6) of the measuring device (1) as determined by the spacers (4) forms the reference surface for measuring the spacing of the individual vertebrae and the measuring sensors (7) consist of a support (13) which can be fixedly clamped to the guide rail (8), of a measuring head (14) which can be displaced on the support (13) transversely with respect to the attachment surface (6) against the force of a return spring (17), and of a displacement sensor (16) for the displacement path of the measuring head (14).

2. Device as claimed in claim 1, **characterised in that** the guide rail (8) is allocated a position transducer (21) for the measuring sensors (7).

3. Device as claimed in claim 1 or 2, **characterised in that** the measuring sensors (7) or the position transducer (21) can be connected to an evaluating circuit which is connected to a display and output unit.

## Revendications

1. Dispositif pour détecter des déplacements de vertèbres, avec un dispositif de mesure (1), comprenant une glissière de guidage (8) pour des sondes de mesure (7) s'étendant transversalement par rapport la glissière de guidage (8), montées de façon réglable en direction de la glissière de guidage (8), pour la mesure d'espacement de la colonne vertébrale d'un patient par rapport à une surface de référence déterminée par le dispositif de mesure (1), **caractérisé en ce que** le dispositif de mesure (1) présente des supports d'espacement (4), pour le placement du dispositif de mesure (1) sur la colonne vertébrale dans la zone dorsale du patient, **en ce que** la surface de pose (6), déterminée par les supports d'espacement (4), du dispositif de mesure (1) forme la surface de référence pour la mesure d'espacement des différentes vertèbres, et **en ce que** les sondes de mesure (7) sont composées d'un support (13) susceptible d'être bloqué par serrage sur la glissière de guidage (8), d'une tête de mesure (14) déplaçable sur le support (13), transversalement par rapport à la surface de pose (6) à l'encontre de la force d'un ressort de rappel (17), et d'un capteur de déplacement (16) pour appréhender la course de déplacement de la tête de mesure (14).

2. Dispositif selon la revendication 1, **caractérisé en ce qu'**un capteur de position (21) pour les sondes de mesure (7) est associé à la glissière de guidage (8).

3. Dispositif selon la revendication 1 ou 2, **caractérisé en ce que** les sondes de mesure (7) ou le capteur de position (21) est/sont susceptible(s) d'être raccordé(s) à un circuit d'évaluation relié à une unité d'affichage et d'édition.
